# EUROPÄISCHE PATENTSCHRIFT

(11) **EP 0 730 848 B1**
(45) Veröffentlichungstag und Bekanntmachung des Hinweises auf die Patenterteilung: **12.04.2000**
(21) Anmeldenummer: 96102401.5
(22) Anmeldetag: 17.02.1996
(51) Int. Cl.: A61F 2/00

(54) **Stent zum Anordnen in einer Körperröhre**
Stent for placement in a body tube
Pilatateur pour placement dans un tube corporel

(30) Priorität: 06.03.1995 DE 19508805
(43) Veröffentlichungstag der Anmeldung: 11.09.1996
(73) Patentinhaber: WILLY RÜSCH AG, D-71394 Kernen (DE)
(72) Erfinder: Freitag, Lutz Dr, D-58675 Hemer (DE)
(74) Vertreter: Ksoll, Peter, Dr.-Ing.

(56) Entgegenhaltungen:
- EP-A- 0 626 153
- WO-A-94/03127
- WO-A-95/31945
- DE-A- 19 524 653

## Beschreibung

Die Erfindung betrifft einerseits einen Stent zum Anordnen in einer Körperröhre gemäß den Merkmalen im Oberbegriff des Anspruchs 1.

Stents sind hohlzylindrische Platzhalter die operativ, percutan oder endoskopisch implantiert werden, um tubuläre Körperröhren, wie beispielsweise Luftröhre, Bronchien, Speiseröhre, Gallengänge, Harnwege, Blutgefäße und ähnliche im Körper offenzuhalten.

Derartige Stents müssen einer äußeren Kompression, welche beispielsweise durch einen Tumor oder einen Lymphknoten hervorgerufen wird oder einer Gefäßstriktur infolge von Verätzung, Verkalkung oder Vernarbung, mit ihrer Rückstellkraft widerstehen.

Zum Offenhalten von Stenosen sind Stents unterschiedlicher Konfiguration und Ausbildung bekannt. Es gibt Kunststoff-, Metall- und Hybridkonstruktionen. Viele haben fixierte Enddurchmesser und sind selbstexpandierend (DE-GM 91 16 881 oder DE-OS 42 40 177).

Andere Ausführungsformen lassen sich durch geeignete Mittel, z. B. Ballons oder Spreizer, im Durchmesser verändern und der jeweiligen anatomischen Situation anpassen. Ein solcher Stent ist aus der US-PS 52 01 901 bekannt.

Weiterhin gehören zum Beispiel durch die DE-OS 42 19 949 oder die DE-OS 43 01 181 Stents aus einer sogenannten Formgedächtnislegierung zum Stand der Technik. Eine Formgedächtnislegierung ist beispielsweise Nitinol. Dieses weist zwei abgrenzbare Zustände auf, die temperaturabhängig eintreten. Nach Vorbehandlung ist Nitinol im kalten Zustand martensitisch, d. h. plastisch verformbar, ohne eine relevante elastische Rückstellkraft. Bei Erwärmung geht es in einen austenitischen, elastischen Zustand über.

Die Formgedächtniseigenschaft wird zur Selbstentfaltung bei unterschiedlichen Stents ausgenutzt.

Die Rückstellkraft, d. h. die Kraft mit der der Stent einer Kompression entgegenwirkt, hängt von der Konstruktion und der Drahtstärke ab.

Aus der EP-A-0 626 153 ist ein Stent bekannt mit einem Stützgerüst aus einem durchgehenden Draht, welcher aus einem Material mit einer Formgedächtnisfunktion besteht. Bei diesem Stent sind einzelne Längenabschnitte in Bezug auf die temperaturabhängigen Umwandlungsbedingungen unterschiedlich. Damit sollen insbesondere die Endbereiche des Stents gegenüber dem Mittelbereich in Bezug auf die Umwandlungsbedingungen unterschiedlich gestaltet werden. Da der Stent aus einem durchgehenden wendelförmigen Draht gebildet ist, ist es schwierig, die temperaturabhängigen Umwandlungsbedingungen des Stents über seine Länge unterschiedlich verteilt zu gestalten. Auch ermöglicht der bekannte Stent nur eine zeitversetzte Entfaltung, indem einzelne Bereiche der Wicklung ihre Form nacheinander ändern. Insgesamt ergibt sich eine gleichmäßige Aufweitung des Stents über die gesamte Länge. Eine Variation der Rückstellkräfte zur medizinischen Anpassung an einen Krankheits- bzw. Behandlungsverlauf ist nicht möglich.

Es gibt weiterhin Stents aus thermoelastischen Kunststoffen, wie Polyurethan, bei denen die Rückstellkraft von der Materialstärke abhängt.

Die bekannten Stents erfüllen je nach Ausführungsform und Anwendungsfall ihre Funktion zufriedenstellend. Dennoch ist es wünschenswert, die Rückstellkraft eines Stents in vivo, d. h. nach Einlage in den Körper, verändern zu können, um so eine Anpassung auf veränderte Zustände vorzusehen, die sich beispielsweise durch ein Tumorwachstum ergeben.

Der Erfindung liegt ausgehend von dem im Oberbegriff des Anspruchs 1 beschriebenen Stent die Aufgabe zugrunde, einen Stent, bei dem die Rückstellkraft im Körper verändert werden kann, anwendungstechnisch zu verbessern.

Die Lösung der Aufgabe besteht in den in den Kennzeichen des Anspruchs 1 aufgeführten Merkmalen.

Kernpunkt der Erfindung bildet die Maßnahme im Stützgerüst eines Stents Drähte mit unterschiedlicher Formgedächtnisfunktion hinsichtlich ihrer Umwandlungstemperaturen zu verwenden und diese in einen Mantel aus einem Memory-Elastomer einzubetten. Legierungen mit Formgedächtnisfunktion werden meist aus einer Kombination der beiden Metalle Nickel und Titan gebildet (Nitinol). Dieses Material hat bei einer tiefen Temperatur eine komprimierte Struktur. Es dehnt sich jedoch bei Überschreiten einer Grenztemperatur aus. Die jeweils gewünschten Grenzbedingungen können durch eine entsprechende Wahl der Legierungskomponenten eingestellt werden.

Ein solcher Draht besitzt ein Hystereseverhalten, d.h. er geht bei einer definierten ersten Umwandlungstemperatur vom martensitischen in den austenitischen Zustand über. Diesen Zustand hält der Draht so lange bei, bis seine Temperatur unter einem zweiten ebenfalls definierten Umwandlungspunkt abgekühlt wird. Dann geht er wieder in weichen, verformbaren, martensitischen Zustand zurück. Dieser Vorgang ist beliebig wiederholbar. Zwischen dem ersten oder oberen Umwandlungspunkt/-temperatur und dem zweiten unteren Umwandlungspunkt besteht eine Temperaturdifferenz von mehreren Grad. Diese wird als Hysterese bezeichnet.

Erfindungsgemäß werden Drähte im Stützgerüst angeordnet, welche bei unterschiedlichen Temperaturen vom martensitischen in den austenitischen Zustand übergehen. Da hierbei eine Hysterese von mehreren Grad auftritt, kann ein Stützgerüst hergestellt werden, welches z.B. durch Applikation von Wärme im physiologischen Bereich in verschiedene Rückstellkräfte versetzt werden kann. Auch ist es möglich, die geometrische Gestalt eines Stents im Ganzen oder nur bereichsweise zu verändern. So kann man einen Stent bereitstellen, der sich an seinen Enden stärker ausformt als in seinem mittleren Bereich. Damit kann die Festlegung eines Stents in einer Körperröhre unterstützt werden.

Das Stützgerüst ist in einem Mantel aus einem Memory-Elastomer eingebettet. Dies ist ist ein temperaturabhängiges Elastomer, welches in kaltem Zustand hart und klein ist.

Folglich kann der Stent gut in der Körperröhre plaziert werden. Erst bei Körpertemperatur bzw. geringfügig unterhalb kommt es zur Expansion des Stents.

Gemäß den Merkmalen des Anspruchs 2 ist das Stützgerüst in mindestens zwei Längenabschnitte mit jeweils eine unterschiedliche Formgedächtnisfunktion aufweisenden Drähten gegliedert.

Je nach gewünschten Eigenschaften, die ein Stent aufweisen soll, wird die Anordnung der Drähte im Stützgerüst ausgewählt. So können Drähte, die jeweils eine unterschiedliche Formgedächtnisfunktion aufweisen, im Stützgerüst abwechselnd angeordnet sein. Es ist aber auch möglich, sie in Gruppen zusammenzufassen.

Damit kann ein Stent nur in einem bestimmten Längenbereich versteift bzw. zusätzlich versteift werden.

So ist es von Vorteil, eine Speiseröhrenprothese (Stent) nur in dem Längenbereich zu versteifen, in dem ein Tumor sitzt. An anderen Stellen aber eine Peristaltik (Schluckakt) zuzulassen. Verändert sich der Tumor durch Wachstum, so daß er einen längeren Speiseröhrenabschnitt beeinträchtigt, kann dann ein entsprechend längerer Abschnitt der Prothese (Stent) versteift und/oder dieser Abschnitt mit einer höheren Rückstellkraft ausgerüstet werden können. Bildet sich der Tumor jedoch zurück, wäre es wiederum wünschenswert, die Rückstellkraft zu vermindern.

Ein Stent kann aus alternierenden Reihen von Drähten aus Nitinol bestehen, wobei die einen Drähte bei 35 °C und die anderen Drähte bei 41 °C in den austenitischen Zustand übergehen. Bei Raumtemperatur ist der Stent klein, plastisch und unelastisch. Nach Einlage in den Körper erwärmt der Stent auf die Körpertemperatur von ca. 37 °C. Er entfaltet sich dabei durch die Rückstellkräfte der ersten Nitinoldrähte. Bei Bedarf können auch die anderen Nitinoldrähte durch Applikation von Wärme umgestellt werden, so daß sie in den austenitischen Zustand übergehen. Damit verdoppelt sich die Rückstellkraft des gesamten Stents.

Die Applikation von Wärme kann durch einen Ballon mit warmem Wasser, aber auch durch elektrischen Strom oder Mikrowellen erfolgen. Erst wenn Kälte bis unter den Hysteresepunkt zugefügt wird, z.B. durch Applikation von Eiswasser, so daß eine Wärmeabfuhr erfolgt, sinkt die Rückstellkraft wieder.

Der Stent kann im Körper verbleiben und bei Bedarf wieder entfaltet werden. Ein solcher Stent kann aber auch auf einfache Weise wieder aus dem Körper entfernt werden.

Nach den Merkmalen des Anspruchs 3 sind in Umfangsrichtung des Stützgerüstes Drähte mit unterschiedlicher Formgedächtnisfunktion verteilt vorgesehen. So kann z.B. ein Stent aus drei verschiedenen Nitinolen hergestellt werden. Mit einem durchspülten Ballonkatheter, der vorzugsweise über eine Temperaturregelung verfügt, ist es dann möglich, an beliebiger Stelle des Stents die Rückstellkraft in drei Abstufungen einzustellen. Durch Kälteapplikation ist dies vollständig reversibel.

In einem Stent können beliebige Konfigurationen des Stützgerüstes vorgesehen sein. Die Drähte können im Stützgerüst unverknüpft oder aber in geeigneter Weise untereinander verbunden sein. Bei den Drähten, die eine definierte Umwandlungstemperatur und ein Hystereseverhalten aufweisen, kann es sich um Metallegierungen handeln (Anspruch 4). Die Drähte können aber auch aus Kunststoff bestehen (Anspruch 5).

Das Stützgerüst kann auch aus einzelnen klammerartigen Drahtspangen aufgebaut sein, welche hintereinander angeordnet sind. Solche Klammerspangen umschließen den Umfang des Stents teilweise. Vorteilhaft sind sie zueinander versetzt angeordnet. Die einzelnen Klammerspangen bestehen aus unterschiedlichen Nitinolen und sind in Silikon eingegossen.

Zur Veränderung der Konfiguration eines in einer Körperröhre plazierten Stents ist dieser mindestens bereichsweise durch eine gezielte Wärme- bzw. Kälteapplikation hinsichtlich der Rückstellkraft und/oder seiner geometrischen Gestalt beeinflußbar. So ist es möglich, mit der medizinisch erforderlichen Präzision genau auf die Bereiche des Stents einzuwirken, in dem eine Umwandlung erfolgen soll.

Vorzugsweise kommt für die Wärme- bzw. Kälteapplikation ein Ballonkatheter mit einer Temperaturkontrolle und/oder -steuerung zum Einsatz. Der Ballonkatheter ist so ausgebildet, daß er über seine Länge Zonen veränderbarer Temperatur aufweist. Auf diese Weise kann eine gezielte Wärme- bzw. Kälteapplikation auf Bereiche des Stents vorgenommen werden.

Die Erfindung ist nachfolgend anhand von in den Zeichnungen dargestellten Ausführungsbeispielen näher beschrieben. Es zeigen:
- Figur 1: einen abgewickelten Ausschnitt aus einem Stent;
- Figur 2: den abgewickelten Ausschnitt einer weiteren Alternative eines Stents;
- Figur 3: eine weitere Ausführungsform eines Stents.

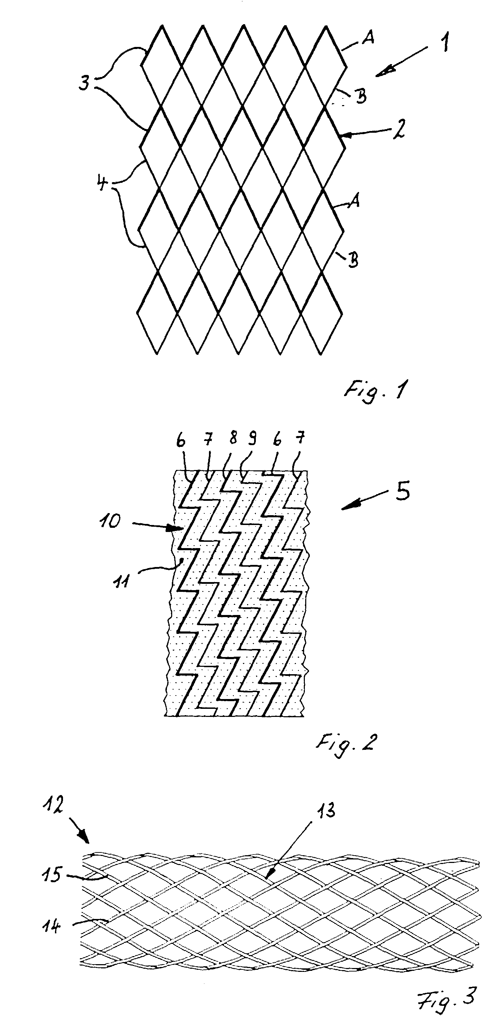

Die Figur 1 zeigt einen abgewickelten Ausschnitt aus einem Stent 1 mit einem Stützgerüst 2, welches aus zickzackförmig angeordneten Drähten 3 und 4 aufgebaut ist.

Die Drähte 3 bestehen aus einem Nitinol mit Hystereseverhalten und einer definierten Umwandlungstemperatur von 35 °C, wohingegen die Drähte 4 aus einem Nitinol mit einer Umwandlungstemperatur von 40 °C besteht. Auf diese Weise werden im Stent 1 einzelne Ringe A und B mit unterschiedlicher Formgedächtnisfunktion gebildet.

Die Ringe A und B sind in Figur 1 durch unterschiedlich dicke Linien angedeutet.

Jeweils abhängig von der Umwandlungstemperatur gehen die Ringe A und B in den austenitischen Zustand über. Bei Raumtemperatur ist der Stent 1 klein und plastisch jedoch unelastisch. Nach dem Plazieren in eine Körperröhre entfaltet sich der Stent 1 bei Körpertemperatur durch den Zustandswechsel der Ringe A. Die Ringe A halten dann ihren Zustand bei.

Bei Bedarf können auch die Ringe B entfaltet werden. Dies erfolgt durch die Applikation von Wärme. Hierdurch verdoppelt sich die Rückstellkraft des gesamten Stents 1.

Eine Applikation von Wärme kann auch gezielt auf nur einzelne Ringe B vorgenommen werden, um eine Aufweitung der Ringe B nur in diesem Bereich vorzunehmen.

In der Figur 2 ist der Ausschnitt aus einem Stent 5 dargestellt, bei dem Drähte 6-9 mit unterschiedlicher Formgedächtnisfunktion in Umfangsrichtung des Stützgerüstes 10 vorgesehen sind. Das Stützgerüst 10 ist in einen Mantel 11 aus einem Elastomer eingebettet.

Die Drähte 6-9 bestehen jeweils aus einer unterschiedlichen Legierung, deren Umwandlungstemperatur bei 35 °C, 38 °C, 40 °C bzw. 42 °C liegt und die ein Hystereseverhalten aufweisen. Auf diese Weise ist es möglich, die Rückstellkraft in vier Abstufungen einzustellen. Dies geschieht durch gezielte Wärmeapplikation. Durch Kälteapplikation ist der Vorgang vollständig reversibel.

Der aus der Figur 3 ersichtliche Stent verfügt über ein geflochtenes Stützgerüst 13.

Das Stützgerüst 13 besteht aus Kunststoffdrähten 14, 15 mit Formgedächtnisfunktion. Auch die Kunststoffdrähte 14, 15 verfügen über eine definierte Umwandlungstemperatur und zeigen ein Hystereseverhalten.

## Patentansprüche

1. Stent (1, 5, 12) zum Anordnen in einer Körperröhre mit einem flexiblen Stützgerüst (2, 10, 13) aus Drähten, welche aus einem Material mit einer Formgedächtnisfunktion bestehen, wobei das Stützgerüst (2, 10, 13) Drähte (3, 4, 6-9, 14, 15) mit unterschiedlicher Formgedächtnisfunktion hinsichtlich ihrer Umwandlungstemperaturen aufweist, dadurch gekennzeichnet, daß das Stützgerüst in einen Mantel (11) aus einem Memory-Elastomer eingebettet ist.

2. Stent (1) nach Anspruch 1, **dadurch gekennzeichnet**, dass das Stützgerüst (2) in mindestens zwei Längenabschnitte (A, B) mit jeweils eine unterschiedliche Formgedächtnisfunktion aufweisenden Drähten gegliedert sind.

3. Stent (2, 12) nach Anspruch 1, **dadurch gekennzeichnet**, dass in Umfangsrichtung des Stützgerüstes (10, 13) Drähte (6-9, 14, 15) mit unterschiedlicher Formgedächtnisfunktion verteilt vorgesehen sind.

4. Stent (1, 5) nach einem der Ansprüche 1 bis 3, **dadurch gekennzeichnet**, dass die Drähte (3, 4, 6-9) aus einer eine definierte Umwandlungstemperatur und ein Hystereseverhalten aufweisenden Formgedächtnislegierung, insbesondere Nitinol, bestehen.

5. Stent (12) nach einem der Ansprüche 1 bis 3, **dadurch gekennzeichnet**, dass die Drähte (14, 15) aus einem eine definierte Umwandlungstemperatur und ein Hystereseverhalten aufweisenden Formgedächtnis-Kunststoff bestehen.

## Claims

1. A stent (1, 5, 12) for disposing in a body tube and comprising a flexible supporting frame (2, 10, 13) of wires made of a material having a shape memory function wherein the supporting frame (2, 10, 13) comprises wires (3, 4, 6 - 9, 14, 15) having different shape memory functions with regard to their conversion temperatures, characterised in that the supporting frame is embedded in a sleeve (11) made of a memory elastomer.

2. A stent (1) according to claim 1, characterised in that the supporting frame (2) is divided into at least two longitudinal portions (A, B) each of wires having a different shape memory function.

3. A stent (2, 12) according to claim 1, characterised in that wires (6 - 9, 14, 15) having different shape memory functions are distributed in the peripheral direction of the supporting frame (10, 13).

4. A stent (1, 5) according to any of claims 1 to 3, characterised in that the wires (3, 4, 6 - 9) are made of a shape memory alloy, particularly Nitinol, having a defined conversion temperature and hysteresis properties.

5. A stent (12) according to any of claims 1 to 3, characterised in that the wires (14, 15) are made of a shape memory plastic having a defined conversion temperature and hysteresis properties.

## Revendications

1. Extenseur ou dilatateur (stent) (1,5,12) destiné à être placé dans un organe corporel tubulaire comportant une structure de support souple (2,10,13) réalisé à partir de fils qui sont constitués par un matériau ayant une fonction de mémoire de forme, la structure de support (2,10,13) présentant des fils (3,4,6-9,14,15) ayant une fonction de mémoire de forme différentielle à l'égard de ses températures de transformation, caractérisé en ce que la structure de support est enrobée dans une enveloppe (11) réalisée en élastomère mémoire.

2. Extenseur ou dilatateur (stent) (1) selon la revendication 1, caractérisé en ce que la structure de support (2) dans au moins deux sections longitudinales (A,B) comporte chaque fois des fils présentant une fonction de mémoire de forme différentielle.

3. Extenseur ou dilatateur (stent) (2,12) selon la revendication 1, caractérisé en ce que dans la direction périphérique de la structure de support (10,13) sont répartis les fils (6-9, 14,15) ayant une fonction de mémoire de forme différentielle.

4. Extenseur ou dilatateur (stent) (1,5) selon l'une des revendications 1 à 3, caractérisé en ce que les fils (3,4,6-9) sont constitués par un alliage de mémoire de forme en particulier du nitinol ayant un comportement d'hystérésis et une température de transformation définie.

5. Extenseur ou dilatateur (stent) (12) selon l'une des revendications 1 à 3, caractérisé en ce que les fils (14,15) sont constitués par une matière synthétique à mémoire de forme ayant un comportement d'hystérésis et une température de transformation définie.
